(19) Europäisches Patentamt / European Patent Office / Office européen des brevets

(11) **EP 3 568 196 B1**

(12) **EUROPEAN PATENT SPECIFICATION**

(45) Date of publication and mention of the grant of the patent:
**29.11.2023 Bulletin 2023/48**

(21) Application number: **18712707.1**

(22) Date of filing: **09.01.2018**

(51) International Patent Classification (IPC):
**A61N 1/36** (2006.01)

(52) Cooperative Patent Classification (CPC):
**A61N 1/36146**

(86) International application number:
**PCT/US2018/012930**

(87) International publication number:
**WO 2018/132367 (19.07.2018 Gazette 2018/29)**

(54) **PATTERNED STIMULATION FOR DEEP BRAIN STIMULATION**

STRUKTURIERTE STIMULATION ZUR TIEFENHIRNSTIMULATION

STIMULATION À MOTIFS POUR STIMULATION CÉRÉBRALE PROFONDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.01.2017 US 201762444446 P**

(43) Date of publication of application:
**20.11.2019 Bulletin 2019/47**

(73) Proprietor: **Boston Scientific Neuromodulation Corporation**
**Valencia, CA 91355 (US)**

(72) Inventor: **BOKIL, Hemant**
**Santa Monica, California 90402 (US)**

(74) Representative: **Vossius & Partner**
**Patentanwälte Rechtsanwälte mbB**
**Siebertstrasse 3**
**81675 München (DE)**

(56) References cited:
DE-A1-102012 002 437      US-A1- 2009 254 144
US-A1- 2012 095 524        US-A1- 2014 277 281

**Description**

TECHNICAL FIELD

This document relates generally to medical devices and more particularly to a system for neurostimulation.

BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device can be used to program the implantable neurostimulator with stimulation parameters controlling the delivery of the neurostimulation energy.

In one example, the neurostimulation energy is delivered in the form of electrical neurostimulation pulses. The delivery is controlled using stimulation parameters that specify spatial (where to stimulate), temporal (when to stimulate), and informational (patterns of pulses directing the nervous system to respond as desired) aspects of a pattern of neurostimulation pulses. Many current neurostimulation systems are programmed to deliver periodic pulses with one or a few uniform waveforms continuously or in bursts. However, the human nervous systems use neural signals having much more sophisticated patterns to communicate various types of information, including sensations of pain, pressure, temperature, etc. US 2012/0095524 A1 discloses a method for counteracting seizure events in a mammalian brain, the method comprising applying an electrical stimulus to the brain, the electrical stimulus being pulsatile and comprising pulses forming a pulse train. In order to effectively "desynchronize" the neural activity patterns in the brain, the pulse train can be at a frequency greater than substantially 300 Hz and at a duty cycle greater than substantially 20%, the pulse train can have an inconstant inter pulse interval such that the pulse rate is not constant throughout the pulse train, and the pulses can have a pulse width greater than substantially 300 µsec. A further neurostimulator is disclosed in DE102012002437 A1.

The present inventor has recognized a need for improvement in the electrical neurostimulation provided by medical devices.

SUMMARY

Electrical neurostimulation energy can be delivered in the form of electrical neurostimulation pulses. More recently, some research has shown that there may be an advantage to temporally patterned stimulation of the neurons of different neuron subgroups. This type of stimulation may be difficult or impossible for a clinician to program into medical devices currently available.

The present invention provides a medical device according to claim 1.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

Figure 1 is an illustration of portions of an example of an electrical stimulation system.
Figure 2 is a schematic side view of an example of an electrical stimulation lead.
Figures 3A-3H are illustrations of different embodiments of leads with segmented electrodes.
Figure 4 is a block diagram of portions of an example of a medical device for providing neurostimulation.
Figure 5 is an illustration of an example of neurostimulation pulses that include bursts of pulses.
Figure 6 is an illustration of an example of delivery of electrical neurostimulation energy using multiple electrodes.

Figure 7 is a graph showing an example of changing the inter-burst frequency during neurostimulation.

Figure 8 is a graph showing an example of changing the intra-burst frequency during neurostimulation.

Figure 9 is an example of neurostimulation that includes frequency modulation of the amplitude of the stimulation pulses.

Figure 10 is an illustration of an example of an electrical neurostimulation signal waveform.

Figure 11 is an illustration of an example of a pulse train generated using a Poisson process.

DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the spirit and scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

This document discusses devices, systems and methods for programming and delivering electrical neurostimulation to a patient or subject. Advancements in neuroscience and neurostimulation research have led to a demand for delivering complex patterns of neurostimulation energy for various types of therapies. The present system may be implemented using a combination of hardware and software designed to apply any neurostimulation (neuromodulation) therapy, including but not being limited to SCS, DBS, PNS, FES, and Vagus Nerve Stimulation (VNS) therapies.

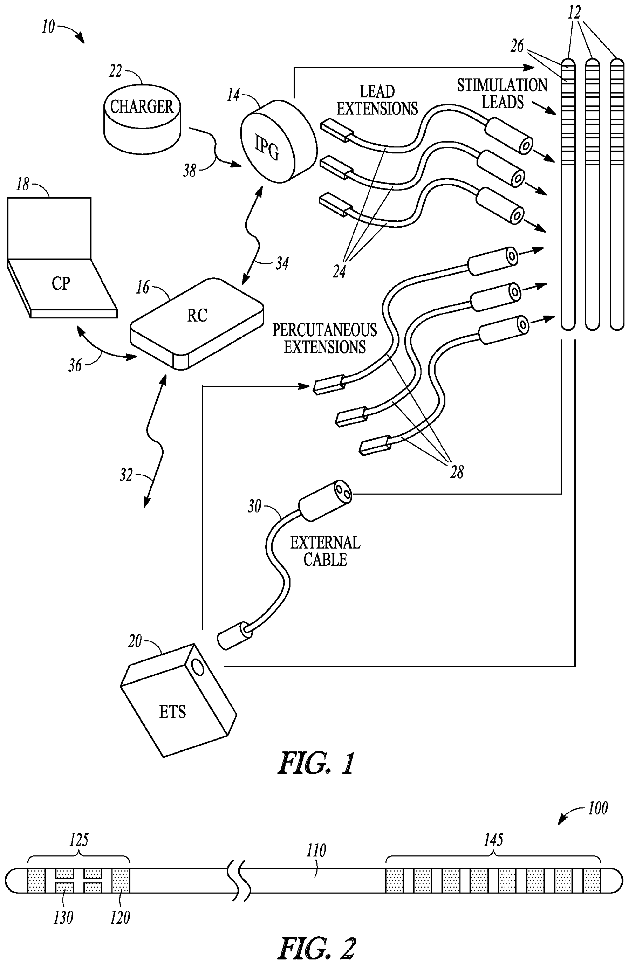

Figure 1 is an illustration of portions of an embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22. The IPG 14 can optionally be physically connected via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The IPG 14 can be implanted into a patient's body, for example, below the patient's clavicle area or within the patient's buttocks or abdominal cavity. The implantable pulse generator can have multiple stimulation channels (e.g., 8 or 16) which may be independently programmable to control the magnitude of the current stimulus from each channel. The IPG 14 can have one, two, three, four, or more connector ports, for receiving the terminals of the leads 12.

The ETS 20 may also be physically connected, optionally via the percutaneous lead extensions 28 and external cable 30, to the stimulation leads 12. The ETS 20, which may have similar pulse generation circuitry as the IPG 14, can also deliver electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a wireless communications link 32. Once the IPG 14 and neurostimulation leads 12 are implanted, the RC 16 may be used to telemetrically communicate with or control the IPG 14 via communications link 34. The communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions. The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18).

For purposes of brevity, the details of the RC 16, CP 18, ETS 20, and external charger 22 will not be further described herein. Details of exemplary embodiments of these devices are disclosed in U.S. Pat. No. 6,895,280, which is incorporated herein by reference. Other embodiments of electrical stimulation systems can be found at U.S. Patents Nos. 6,181 ,969; 6,516,227; 6,609,029; 6,609,032; 6,741,892; 7,949,395; 7,244,150; 7,672,734; 7,761,165; 7,974,706; 8,175,710; 8,224,450; 8,364,278; and 8,700,178, all of which are incorporated herein by reference.

Figure 2 is a schematic side view of an embodiment of an electrical stimulation lead. Figure 2 illustrates a lead 110 with electrodes 125 disposed at least partially about a circumference of the lead 110 along a distal end portion of the lead and terminals 145 disposed along a proximal end portion of the lead. The lead 110 can be implanted near or within the desired portion of the body to be stimulated (e.g., the brain, spinal cord, or other body organs or tissues). In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of a stylet (not shown). The lead 110 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 110, advance the lead 110, retract the lead 110, or rotate the lead 110.

In some embodiments, measurement devices coupled to the muscles or other tissues stimulated by the target neurons, or a unit responsive to the patient or clinician, can be coupled to the implantable pulse generator or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 110 for deep brain stimulation can include stimulation electrodes, recording electrodes, or both. In at least some embodiments, the lead 110 is rotatable so that the stimulation electrodes can be aligned with the target neurons after the neurons have been located using the recording electrodes. Stimulation electrodes may be disposed on the circumference of the lead 110 to stimulate the target neurons. Stimulation electrodes may be ring-shaped so that current projects from each electrode equally in every direction from the position of the electrode along a length of the lead 110. In the embodiment of Figure 2, two of the electrodes 120 are ring electrodes 120. Ring electrodes typically do not enable stimulus current to be directed from only a limited angular range around of the lead. Segmented electrodes 130, however, can be used to direct stimulus current to a selected angular range around the lead. When segmented electrodes are used in conjunction with an implantable pulse generator that delivers constant current stimulus, current steering can be achieved to more precisely deliver the stimulus to a position around an axis of the lead (*e.g.*, radial positioning around the axis of the lead). To achieve current steering, segmented electrodes can be utilized in addition to, or as an alternative to, ring electrodes.

The lead 100 includes a lead body 110, terminals 145, and one or more ring electrodes 120 and one or more sets of segmented electrodes 130 (or any other combination of electrodes). The lead body 110 can be formed of a biocompatible, non-conducting material such as, for example, a polymeric material. Suitable polymeric materials include, but are not limited to, silicone, polyurethane, polyurea, polyurethaneurea, polyethylene, or the like. Once implanted in the body, the lead 100 may be in contact with body tissue for extended periods of time. In at least some embodiments, the lead 100 has a cross-sectional diameter of no more than 1.5 millimeters (1.5 mm) and may be in the range of 0.5 to 1. 5 mm. In at least some embodiments, the lead 100 has a length of at least 10 centimeters (10 cm) and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use. Each of the electrodes can either be used or unused (OFF). When the electrode is used, the electrode can be used as an anode or cathode and carry anodic or cathodic current. In some instances, an electrode might be an anode for a period of time and a cathode for a period of time.

Deep brain stimulation leads and other leads may include one or more sets of segmented electrodes. Segmented electrodes may provide for superior current steering than ring electrodes because target structures in deep brain stimulation or other stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue.

Embodiments of leads with segmented electrodes include U.S. Patents Nos. 8,473,061; 8,571,665; and 8,792,993; U.S. Patent Application Publications Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/197375; 2012/0203316; 2012/0203320; 2012/0203321; 2013/0197424; 2013/0197602; 2014/0039587; 2014/0353001 ; 2014/0358208; 2014/0358209; 2014/0358210; 2015/0045864; 2015/0066120; 2015/0018915; 2015/0051681; 2015/0151113; and 2014/0358207.

Any number of segmented electrodes 130 may be disposed on the lead body 110 including, for example, anywhere from one to sixteen or more segmented electrodes 130. It will be understood that any number of segmented electrodes 130 may be disposed along the length of the lead body 110. A segmented electrode 130 typically extends only 75%, 67%, 60%, 50%, 40%, 33%, 25%, 20%, 17%, 15%, or less around the circumference of the lead.

The segmented electrodes 130 may be grouped into sets of segmented electrodes, where each set is disposed around a circumference of the lead 100 at a particular longitudinal portion of the lead 100. The lead 100 may have any number segmented electrodes 130 in a given set of segmented electrodes. The lead 100 may have one, two, three, four, five, six, seven, eight, or more segmented electrodes 130 in a given set. In at least some embodiments, each set of segmented electrodes 130 of the lead 100 contains the same number of segmented electrodes 130. The segmented electrodes 130 disposed on the lead 100 may include a different number of electrodes than at least one other set of segmented electrodes 130 disposed on the lead 100. The segmented electrodes 130 may vary in size and shape. In some embodiments, the segmented electrodes 130 are all of the same size, shape, diameter, width or area or any combination thereof. In some embodiments, the segmented electrodes 130 of each circumferential set (or even all segmented electrodes disposed on the lead 100) may be identical in size and shape.

Each set of segmented electrodes 130 may be disposed around the circumference of the lead body 110 to form a substantially cylindrical shape around the lead body 110. The spacing between individual electrodes of a given set of the segmented electrodes may be the same, or different from, the spacing between individual electrodes of another set of segmented electrodes on the lead 100. In at least some embodiments, equal spaces, gaps or cutouts are disposed between each segmented electrode 130 around the circumference of the lead body 110. In other embodiments, the spaces, gaps or cutouts between the segmented electrodes 130 may differ in size, or cutouts between segmented electrodes 130 may be uniform for a particular set of the segmented electrodes 130 or for all sets of the segmented electrodes 130. The sets of segmented electrodes 130 may be positioned in irregular or regular intervals along a length the lead body 110.

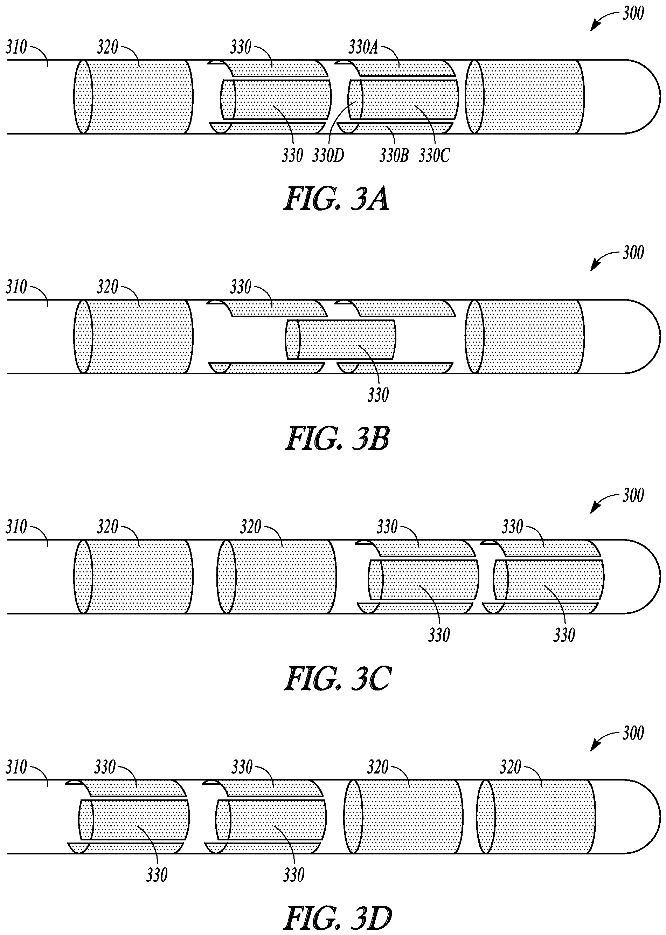

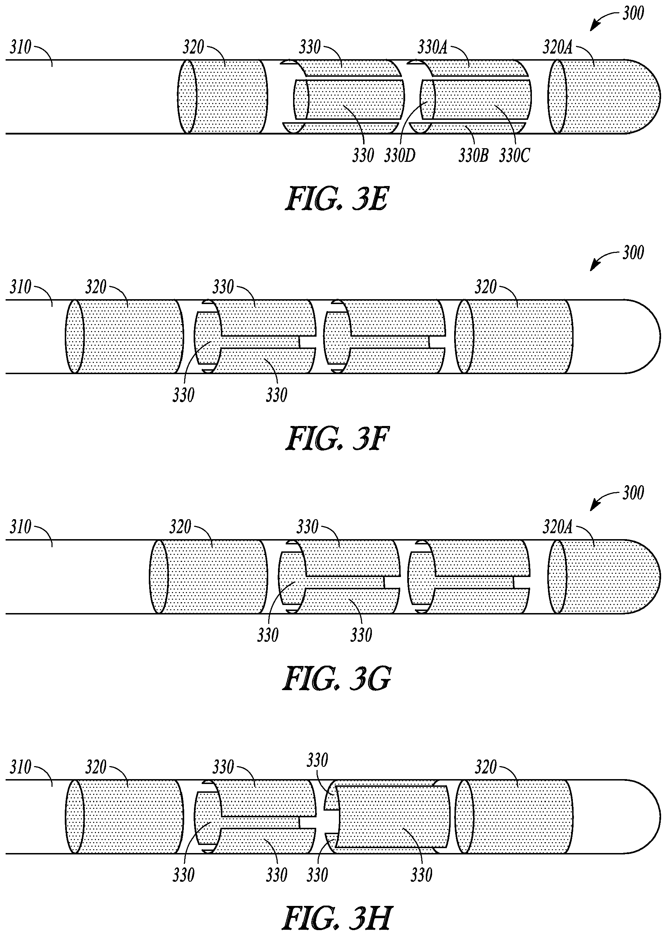

Conductor wires (not shown) that attach to the ring electrodes 120 or segmented electrodes 130 extend along the lead body 110. These conductor wires may extend through the material of the lead 100 or along one or more lumens defined by the lead 100, or both. The conductor wires couple the electrodes 120, 130 to the terminals 145. Figures 3A-3H are illustrations of different embodiments of leads 300 with segmented electrodes 330, optional ring electrodes 320 or tip electrodes 320a, and a lead body 310. The sets of segmented electrodes 330 each include either two (Figure 3B), three (Figures 3E-3H), or four (Figures 3A, 3C, and 3D) or any other number of segmented electrodes including, for example, three, five, six, or more. The sets of segmented electrodes 330 can be aligned with each other (Figures 3A-3G) or staggered (Figure 3H).

When the lead 100 includes both ring electrodes 120 and segmented electrodes 130, the ring electrodes 120 and the segmented electrodes 130 may be arranged in any suitable configuration. For example, when the lead 100 includes two ring electrodes 120 and two sets of segmented electrodes 130, the ring electrodes 120 can flank the two sets of segmented electrodes 130 (see *e.g.*, Figures 2, 3A, and 3E-3H, ring electrodes 320 and segmented electrode 330). Alternately, the two sets of ring electrodes 120 can be disposed proximal to the two sets of segmented electrodes 130 (*see e.g.*, Figure 3C, ring electrodes 320 and segmented electrode 330), or the two sets of ring electrodes 120 can be disposed distal to the two sets of segmented electrodes 130 (*see e.g.*, Figure 3D, ring electrodes 320 and segmented electrode 330). One of the ring electrodes can be a tip electrode (see *e.g.*, tip electrode 320a of Figures 3E and 3G). It will be understood that other configurations are possible as well (e.g., alternating ring and segmented electrodes, or the like).

By varying the location of the segmented electrodes 130, different coverage of the target neurons may be selected. For example, the electrode arrangement of Figure 3C may be useful if the physician anticipates that the neural target will be closer to a distal tip of the lead body 110, while the electrode arrangement of Figure 3D may be useful if the physician anticipates that the neural target will be closer to a proximal end of the lead body 110.

Any combination of ring electrodes 120 and segmented electrodes 130 may be disposed on the lead 100. For example, the lead may include a first ring electrode 120, two sets of segmented electrodes; each set formed of four segmented electrodes 130, and a final ring electrode 120 at the end of the lead. This configuration may simply be referred to as a 1-4-4-1 configuration (Figures 3A and 3E, ring electrodes 320 and segmented electrode 330). It may be useful to refer to the electrodes with this shorthand notation. Thus, the embodiment of Figure 3C may be referred to as a 1-1-4-4 configuration, while the embodiment of Figure 3D may be referred to as a 4-4-1-1 configuration. The embodiments of Figures 3F, 3G, and 3H can be referred to as a 1-3-3-1 configuration. Other electrode configurations include, for example, a 2-2-2-2 configuration, where four sets of segmented electrodes are disposed on the lead, and a 4-4 configuration, where two sets of segmented electrodes, each having four segmented electrodes 130 are disposed on the lead. The 1-3-3-1 electrode configuration of Figures 3F, 3G, and 3H has two sets of segmented electrodes, each set containing three electrodes disposed around the circumference of the lead, flanked by two ring electrodes (Figures 3F and 3H) or a ring electrode and a tip electrode (Figure 3G). In some embodiments, the lead includes 16 electrodes. Possible configurations for a 16-electrode lead include, but are not limited to 4-4-4-4; 8-8; 3-3-3-3-3-1 (and all rearrangements of this configuration); and 2-2-2-2-2-2-2-2.

Any other suitable arrangements of segmented and/or ring electrodes can be used including, but not limited to, those disclosed in U.S. Provisional Patent Application Serial No. 62/113,291 and U.S. Patent Applications Publication Nos. 2012/0197375 and 2015/0045864. As an example, arrangements in which segmented electrodes are arranged helically with respect to each other. One embodiment includes a double helix.

One or more electrical stimulation leads can be implanted in the body of a patient (for example, in the brain or spinal cord of the patient) and used to stimulate surrounding tissue. The lead(s) are coupled to the implantable pulse generator (such as IPG 14 in Figure 1). After implantation, a clinician will program the IPG 14 using the clinician programmer, remote control, or other programming device. According to at least some programming techniques, the clinician enters stimulator parameters for a stimulation program and the stimulation program is used to stimulate the patient. The clinician observes the patient response. In at least some instances, the clinician asks the patient to describe, rate, or otherwise provide information about the effects of the stimulation such as what portion of the body is affected, how strong is the stimulation effect, whether there are side effects or negative effects, and the like.

Electrical neurostimulation can be provided to the tissue targets in a repeated pattern using the electrodes of the stimulation leads. If the pattern is not varied, this is sometimes referred to as tonic stimulation. There may be an advantage to unsynchronized stimulation of the neurons of different neuron subgroups. To affect this unsynchronized stimulation, the electrical neurostimulation energy is provided to electrodes in bursts of pulses in which the burst parameters are varied.

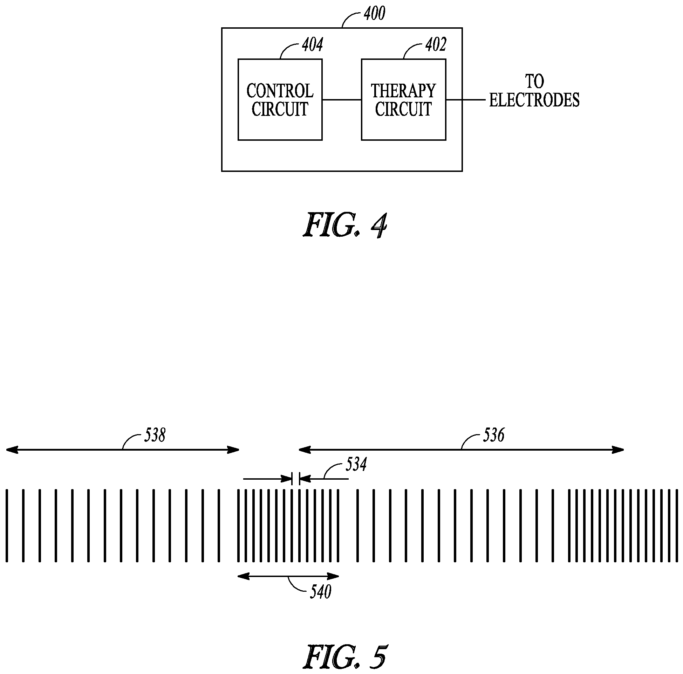

Figure 4 is a block diagram of portions of an embodiment of a medical device 400 for providing neurostimulation. The device 400 includes a therapy circuit 402 and a control circuit 404. The therapy circuit 402 can be operatively coupled to stimulation electrodes such as any of the electrodes described herein and provides or delivers electrical neurostimulation energy to the electrodes. The control circuit 404 can include a processor such as a microprocessor, a digital signal processor, application specific integrated circuit (ASIC), or other type of processor, interpreting or executing instructions in software modules or firmware modules. In some embodiments, the control circuit 404 includes a logic sequencer circuit. A logic sequencer refers to a state machine or other circuit that sequentially steps through a fixed series of steps to perform one or more functions. The steps are typically implemented in hardware or firmware. The control circuit 404 can include other circuits or sub-circuits to perform the functions described. These circuits may include software, hardware, firmware or any combination thereof. Multiple functions can be performed in one or more of the circuits or sub-circuits as desired. For example, the control circuit 404 initiates delivery of bursts of pulses of the electrical neurostimulation energy to the electrodes. The control circuit can include one or more timer sub-circuits to time the activation and deactivation of the therapy circuit 402 to implement the burst timing.

Figure 5 is an illustration of an embodiment of neurostimulation pulses that include bursts of pulses. In the embodiment, the neurostimulation pulses can be delivered according to an intra-burst time period 534 and an inter-burst time period 536. The intra-burst time period is the time between pulses during a burst, and intra-burst frequency is the frequency at which the pulses are delivered. In Figure 5, the pulses are delivered using two frequencies; pulses 538 delivered at a relatively lower frequency that is alternated with a burst of pulses 540 at a relatively higher frequency. In an illustrative example not intended to be limiting, the lower frequency may be 50-200 hertz (Hz) and the higher frequency may be 100-300Hz. In certain variations, only pulses at the burst rate are delivered and no pulses are delivered at the lower frequency between bursts. The time from the beginning of the first burst to the beginning of the second burst is the inter-burst time period. The inter-burst time period can also be viewed in Figure 5 as the time for a cycle of the fast and slow pulses to repeat. Because the frequency is the inverse of the time period, the cycles can be viewed as repeating with an inter-burst frequency. The neurostimulation pulses can be delivered for a running time with a pause between neurostimulation. The control circuit can restart the running time after the duration of the pause.

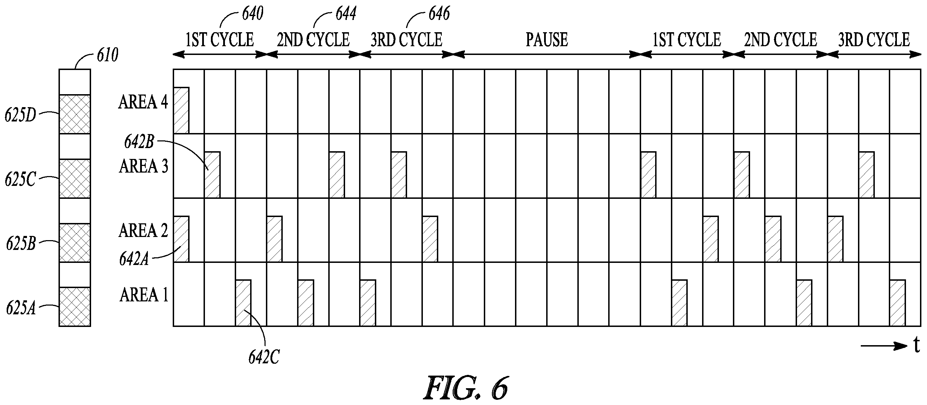

Figure 6 is an illustration of an example of delivery of electrical neurostimulation energy using multiple electrodes. In the example, bursts of pulses are delivered using lead 610 that has four electrodes. The timing of the neurostimulation can be controlled using the control circuit 404 of FIG. 4. The electrodes may be ring electrodes or segmented electrodes. Stimulation is provided to target area 1 using electrode 625A, target area 2 using electrode 625B, and target area 3 using electrode 625C. Electrode 625D may be a reference electrode or may be used to provide stimulation to a target area 4. However, this is only an example, and a stimulation area may not be tied to a unique electrode. Stimulation of a target area can include activation of multiple electrodes. Different percentages of the stimulation can be provided by each of the electrodes

Figure 6 shows that three cycles of neurostimulation are delivered to the patient or subject followed by a pause in therapy. One cycle of neurostimulation includes delivering a burst of pulses to areas 1-3. The first cycle 640 begins with delivery of one or more bursts of multiple pulses 642A delivered to area 2 using electrode 625B. The first cycle of stimulation then proceeds with delivery of one or more bursts of pulses 642B to area 3 using electrode 625C and then a delivery of one or more bursts of pulses 642C to area 1 using electrode 625A.

The first cycle 640 of neurostimulation is followed by a second cycle 644 of neurostimulation. Like the first cycle, the second cycle 644 begins with delivery of one or more bursts of multiple pulses 642A to area 2 using electrode 625B, but differs from the first cycle by delivering the bursts of pulses to area 1 using electrode 625A before delivering the bursts of pulses to area 3 using electrode 625C. Thus, the order of delivering stimulation energy to the electrodes is changed from the first cycle 640. The second cycle 644 is followed by a third cycle of neurostimulation. The third cycle 646 begins with a delivery of one or more bursts of pulses to area 1 using electrode 625A, followed by one or more bursts of pulses to area 3 using electrode 625C, followed by one or more bursts of pulses delivered to area 2 using electrode 625B. Thus, the order of the delivery of stimulation energy to the electrodes in the third cycle is changed from both the first cycle and the second cycle. The first three cycles of neurostimulation are followed by a pause in stimulation, and the pause is followed by a second three cycles of neurostimulation. The duration of the cycles can be the same (e.g., ten seconds) or can be different. It can be seen in the Figure that the order of electrodes used in the stimulation of the second three cycles is different from the order used in the first three cycles.

In some embodiments, the combination of the areas to which neurostimulation is delivered can be changed during the neurostimulation. For instance, the stimulation to area 4 using electrode 625D can be substituted for the stimulation using any of electrodes 625A-625C during any of the cycles in the example. It can be seen in the example of Figure 6 that changing a combination of electrodes used to deliver the bursts of pulses changes positions on the leads (and thereby changes the target area or areas) to which the bursts are delivered. In some embodiments, neurostimulation using electrode 625D can be added during any of the cycles shown in the example of Figure 6. For instance, neurostimulation can be delivered during the first cycle to both area 2 and area 4 using both electrode 625B and electrode 625D. This may result in delivering twice the energy during the first cycle as compared to the other cycles, or the energy may be split between electrodes 625B and 625D. For instance a first fraction of neurostimulation energy of the burst of pulses may be delivered to area 2 using electrode 625B and a second fraction of the neurostimulation energy of the burst of pulses may be delivered to area 4 using electrode 625D during the same cycle. Together the fractions of the neurostimulation energy may equal the energy delivered using only electrode 625C during the second cycle.

Neurostimulation can be delivered using a first combination of the electrodes for a first cycle or cycles of stimulation, and a second combination can be used for a second cycle or set of cycles. This approach can be extended to more electrodes as shown in the lead example of Figure 2 or to multiple leads as shown in the system example of Figure 1. For instance, for an eight electrode lead that delivers neurostimulation to eight target tissue areas A1 through A8, a burst of neurostimulation pulses may be simultaneously delivered to three of the eight target areas and the three target areas used may be circulated among the eight target areas available, such as (A1, A4, A8); (A2, A4, A8); (A1, A2, A3); (A4, A2, A8); (A2, A3, A5); (A8, A6, A2); (A1, A2, A3); (A4, A2, A8); (A4, A7, A8); (A1, A5, A8); (A2, A4, A6); and so on. In some embodiments, repeats of the target areas stimulated are minimized.

Burst parameters may be modified by the controller circuit during the neurostimulation. For instance, the number of cycles delivered between pauses in the neurostimulation therapy and the duration of the pauses may be varied during the neurostimulation. As another example, one or both of the pulse amplitude and the pulse width may be changed from burst-to-burst or changed within the same burst. Further, one or more of the inter-burst time period and intra-burst time period.

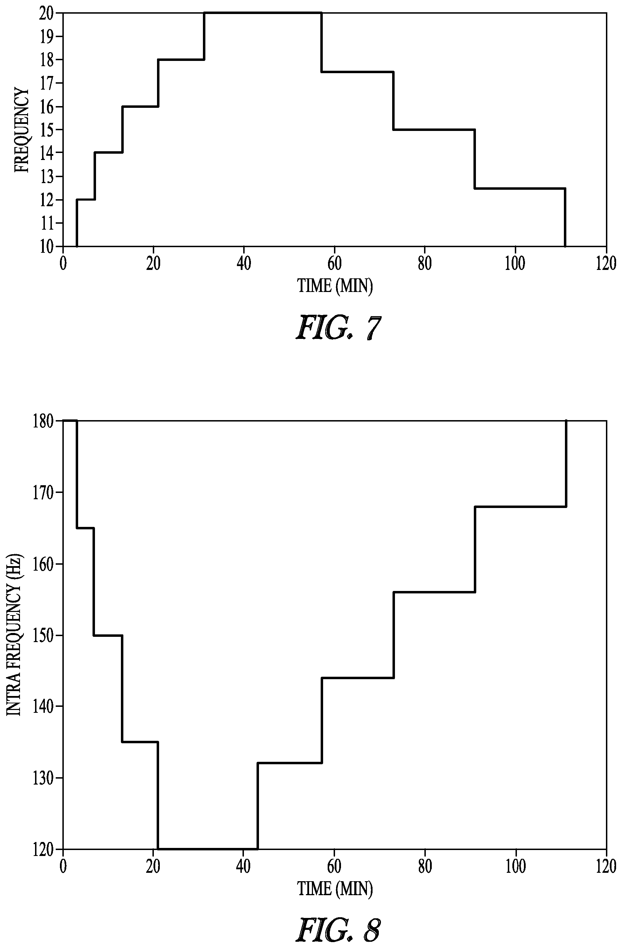

Figure 7 is a graph showing an example of changing the inter-burst frequency during neurostimulation. The vertical axis is the inter-burst frequency and the horizontal axis is time (t) during the neurostimulation. The inter-burst frequency is increased and then decreased during the 120 second (120s) duration shown. At the beginning of the neurostimulation (t = 0s), bursts of neurostimulation are delivered with an inter-burst frequency of 10Hz (or one burst cycle every 0.1s). The inter-burst frequency is increased to 12Hz at $t$ = 3s. The inter-burst frequency continues to be increased to 14Hz, 16Hz, 18Hz, and 20Hz at $t$ = 7s, 13s, 21s, and 31s, respectively. The inter-burst frequency remains at 20Hz until $t$ = 57s, where it is decreased to 17.5Hz. The inter-burst frequency continues to be decreased to 15Hz, 12.5Hz, and 10Hz at $t$ = 73s, 91s, and 111s, respectively. In addition (or in alternative) to changing the inter-burst frequency the intra-burst frequency may be changed during the neurostimulation.

Figure 8 is a graph showing an example of changing the intra-burst frequency during the 120 second neurostimulation of Figure 7. The vertical axis is the intra-burst frequency and the horizontal axis is time ($t$) during the neurostimulation. The intra-burst frequency is decreased and then increased during the time duration shown. At the beginning of the neurostimulation ($t$ = 0s), the intra-burst frequency of the bursts of neurostimulation 1s 180Hz. The intra-burst frequency is decreased to 165Hz, 150Hz, 135Hz, and 120Hz at $t$ = 3s, 7s, 13s, and 21s, respectively. The intra-burst frequency remains at 120Hz until $t$ = 43s, where it is increased to 132Hz. The intra-burst frequency continues to be increased to 144Hz, 156Hz, 168Hz, and 180Hz at $t$ = 57s, 73s, 91s, and 111s, respectively.

In some embodiments, one or both of the inter-burst frequency and the intra-burst frequency are changed in addition to changing the electrodes used to deliver the neurostimulation as shown in the example of Figure 6. In some embodiments, repeats of a combination of the inter-burst frequency and the intra-burst frequency are minimized.

For example, a first burst of electrical pulses to a first electrode using a first intra-burst period and simultaneously deliver a second burst of electrical pulses to a second electrode using a second intra-burst period. In another example, a burst of pulses can be delivered to a first electrode using a first inter-burst period and first intra-burst period and one or both of the inter-burst period and the intra-burst period may be different in the bursts delivered to a second electrode during the neurostimulation.

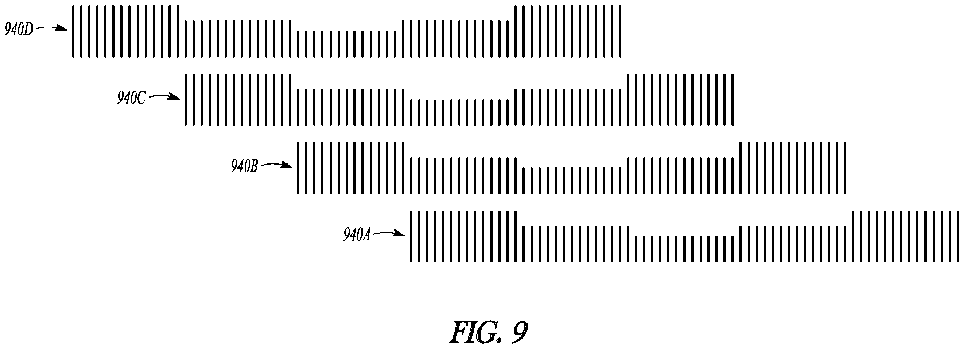

According to some examples, one or both of the modulate amplitude and pulse width of the pulses of the electrical neurostimulation energy is frequency modulated during the neurostimulation. Figure 9 is an embodiment of neurostimulation that includes frequency modulation of the amplitude of the stimulation pulses. Four pulse trains are shown in the Figure. The pulse trains may be delivered using different electrodes of a lead. For instance, pulse train 940D may be delivered using electrode 625D in FIG. 6, pulse train 940C may be delivered using electrode 625C, pulse train 940B may be delivered using electrode 625B, and pulse train 940A may be delivered using electrode 625A. In certain embodiments, the pulse trains may be delivered using different electrodes of different leads. The pulses in the example of Figure 9 are delivered with a high intra-burst frequency and the pulses are amplitude modulated using a low modulation frequency.

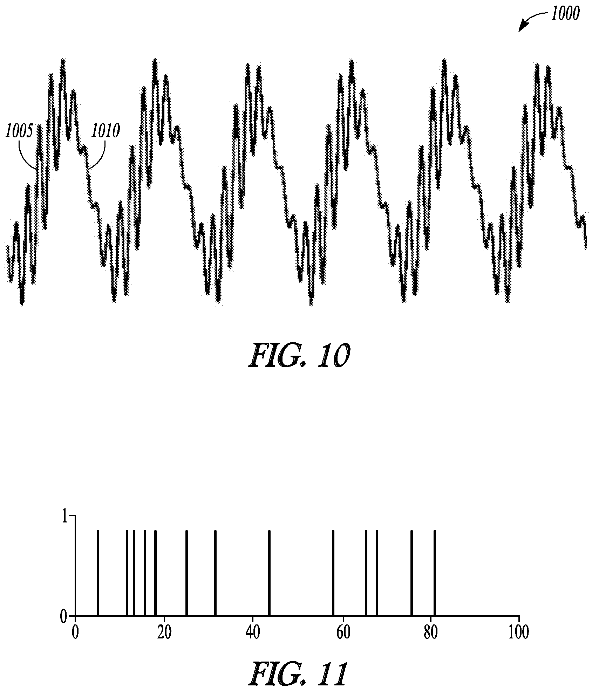

Figure 10 is an illustration of an example of an electrical neurostimulation signal waveform 1000. The neurostimulation signal may be generated by the therapy circuit 405 of Figure 4. The electrical neurostimulation signal includes a lower frequency signal component and a higher frequency signal component imposed on the lower signal frequency component. The higher frequency signal component has a lower amplitude than the lower frequency signal component. In some embodiments, the lower frequency is within the range of 15-25Hz and the higher frequency signal is within the range of 30-90Hz. In the example in Figure 10, the higher frequency signal component has two amplitudes. One amplitude on the first or rising phase 1005 of the lower frequency signal component and a second different amplitude on the falling phase 1010. The amplitude on the falling phase is shown smaller than the first amplitude of the rising phase.

Neurostimulation therapy parameters have been described that include pulse amplitude, pulse width, intra-burst frequency, inter-burst frequency, and run time. The control circuit 410 of Figure 4 may schedule delivery of neurostimulation that cycles through one or more sequences of different inter-burst *frequencies f* (e.g., $f_1, f_2, ..., f_N$) for different run times *t* (e.g., $t_1, t_2, ..., t_N$). To further unsynchronize the neurostimulation, the control circuit 410 may randomize changes in one or more of pulse amplitude, pulse width, intra-burst frequency, inter-burst frequency, and run time during neurostimulation therapy. In some embodiments, the control circuit 410 initiates delivery of pulses using a randomization function in which one or more of pulse amplitude, pulse width, intra-burst frequency, inter-burst frequency, and run time are given a value that is selected randomly.

According to some embodiments, the control circuit 410 initiates delivery of a scheduled pulse according to a probability function, such as by drawing the pulse times from a probability distribution. In some embodiments, the control circuit draws values of one or both of intra-burst frequencies and inter-burst frequencies according to a probability function.

In some embodiments, the probability function is a Poisson process. The probability that a neurostimulation pulse is delivered by the medical device during an interval between time *t* and time *dt* is determined (e.g., using a control circuit) according to $P = Rdt$, where *P* is the pulse event and *R* is a predetermined number. To generate a pulse by simulating a Poisson process, a sequence of uniform random numbers is chosen ($U_1, U_2 ... U_N$) where the numbers have a value between zero and one. A pulse event P can then be generated using:

$$|P| = -log(U_1)/R, -log(U_2)/R \dots -log(U_N)/R,$$

with R being an intra-burst frequency value of 100-200Hz.

A Poisson process can also be simulated by establishing a series of small bins representing a small time duration (e.g., $\Delta t$ = one millisecond (1ms) corresponding to an intra-burst frequency of 1000Hz). A random number z between zero and one (i.e., $0 < z < 1$) is chosen and assigned to each bin. If the number R for a bin is greater than the assigned value z, (or $R\Delta t > z$), generate a pulse event P, otherwise do not generate a pulse event. Figure 11 is an illustration of an example of a pulse train generated using a Poisson process. The horizontal access is time in milliseconds corresponding to the series of bins, and the vertical access is either 0 or 1 with one denoting occurrence of a pulse event.

In some examples, probabilistic bursting of pulses can be implemented using a Poisson process. Multiple pulses can be scheduled for delivery as a burst of pulses and the number of pulses to include in the burst can be determined according to the probability function. For example, pulse events P can be generated using:

$$|P| = -log(U_N)/R_1,$$

as above with $R_1$ being an intra-burst frequency value of 130Hz. A value of an integer N can be determined using probability, e.g., such as by using the probability function

$$P(N) = (R_2{}^N e^{-R2})/N! \, ,$$

where $R_2$ is an inter-burst frequency (e.g., $R_2$ = 1HZ), N pulses *at $R_1$* are generated at the beginning of the inter-burst cycle time.

In some embodiments, stimulation pulses can be delivered according to a probability function that is a Gamma process. In a Gamma process, the probability of a pulse event occurring depends on when the last pulse event occurred. The Gamma pobability distribution can be determined as

$$P(\tau) = (kr)^k \tau^{k-1} e^{-kr\tau}/(k-1)! \ ,$$

where r is an intra-burst frequency (e.g., 100-200Hz), and $k$ = 1, 2, 3, 4, 5. The Gamma process reduces to a Poisson process when k = 1.

In some embodiments, the pulse stimulation is delivered using a probability that a scheduled pulse will be delivered determined using a history of pulse delivery over a time period prior to the scheduled pulse delivery. For example, the probability that a scheduled pulse event is delivered may be determined according to

$$P = e^{-(a0+a1+a2 \ ... \ aN)},$$

where *a0, a1, a2, ... aN* are coefficients. The baseline value of the probability equation is $e^{-(a0)}$ and use or inclusion of *a1*, *a2*, ... or *aN in* determining the probability depends on the past events over the prior specified time period (e.g., 100-150ms).

In an illustrative example intended to be non-limiting, assume the prior time period is 150ms. A series of fifteen bins are assigned to every 10ms of the 150ms period and a coefficient is assigned to each bin (e.g., *a1*, *a2* ... *a15).* If a pulse occurred over the previous time period during that bin, its corresponding coefficient is included in the equation. If a pulse occurred during the previous 10ms *a1* is included in the equation, if a pulse occurred during the previous 10-20ms *a2* is included in the equation, if a pulse occurred during the previous 20-30ms *a3* is included in the equation ... and if a pulse occurred during the previous 140-150ms *a15* is included in the equation.

The probability of pulsing can be enhanced or suppressed relative to the baseline probability $e^{-(a0)}$ by choosing values for the coefficients *a1, a2 ... aN*. For the illustrative example above, if *a0* = -5, *a1- a9* = 0, $\alpha10$ = 5, *a11- a14* = 0, and *a15* = -10, the pulsing probability of a currently scheduled pulse is suppressed when pulsing at 95ms during the prior time period and enhanced when pulsing at 145ms during the prior time period.

In some embodiments, the control circuit schedules pulses using a time interval between pulses and the probability function is used to change the time intervals between successive pulses. As an illustrative example intended to be non-limiting, a series time durations (e.g., $\Delta t$, $2\Delta t$, $3\Delta t$ ..., where $\Delta t$ = 10ms) is clocked or timed. Starting with the first bin, a probability function is used to select a pulse frequency for that bin and pulses are delivered during the first bin using the selected frequency time interval during that bin. Timing then proceeds to the second bin where another pulse frequency is determined according to the probability function and pulses are delivered during the second bin using the frequency time interval selected for that bin. Timing then proceeds to the third bin where the process is repeated, and so on. In some embodiments, the pulse frequency for a bin is selected using a randomization function.

In some embodiments, different tissue targets are stimulated according to different probability during the same neurostimulation. For instance, multiple electrodes can be disposed at multiple tissue targets. The control circuit may schedule delivery of pulses of the electrical neurostimulation energy to a first set of the electrodes and a second set of the electrodes, and initiate delivery of pulses to the first set of electrodes using a first probability function and initiate delivery of pulses to the second set of electrodes using a second probability function that is different from the first probability function. In an illustrative example intended to be non-limiting, the control circuit may randomly determine a value for one or more of an intra-burst frequency, an inter-burst frequency, and a run time for the first set of electrodes, and may determine a value for one or more of an intra-burst frequency, an inter-burst frequency, and a run time for the second set of electrodes according to a Poisson process.

Many different options have been described for providing neurostimulation using the medical device 400 of Figure 4. A user interface (e.g., a user interface of an external RC 16 or CP 18 of Figure 1) can be used for programming of the neurostimulation parameters described. In some embodiments, the user interface includes a graphical user interface (GUI) that displays graphs or representations of neurostimulation (e.g., representations such as those of Figure 6, Figure 7, or Figure 8) with field available for a clinician to enter parameters for the neurostimulation. This can help the user with understanding and interpretation of the programmed neurostimulation.

The several embodiments described herein can provide unsynchronized stimulation of the neurons of different neuron subgroups. Delivery of neurostimulation can also include unsynchronized stimulation to different tissue target areas for decentralizing the location of the stimulation. This allows different neuron subgroups to be stimulated at different times in a non-monotonic fashion.

The embodiments described herein can be methods that are machine or computer-implemented at least in part. Some embodiments may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. Various embodiments are illustrated in the figures above. One or more features from one or more of these embodiments may be combined to form other embodiments.

The above detailed description is intended to be illustrative, and not restrictive. The scope of the invention is defined by the appended claims.

**Claims**

1. A medical device (400) for coupling to a plurality of implantable electrodes, the medical device comprising:

   a therapy circuit (402) configured to deliver electrical neurostimulation energy to the plurality of implantable electrodes (26); and
   a control circuit (404) operatively coupled to the therapy circuit and configured to:

   initiate delivery of bursts of pulses of the electrical neurostimulation energy to a first electrode of the plurality of the implantable electrodes, wherein the bursts of pulses provided to the first electrode include a first inter-burst period between bursts and pulses within a burst provided to the first electrode include a first intra-burst period; and
   initiate delivery of bursts of pulses to a second electrode of the plurality of electrodes , wherein the bursts of pulses provided to the second electrode include a second inter-burst period between bursts; and **characterized in that** the pulses within a burst provided to the second electrode include a second intra-burst period that is less than the first intra-burst period.

2. The medical device of claim 1, further comprising one or more implantable leads (12), wherein the plurality of implantable electrodes are disposed at different positions on the one or more implantable leads; and wherein the first electrode and the second electrode correspond to different positions on the one or more implantable leads to which the bursts are delivered.

3. The medical device of claim 2, wherein the control circuit is further configured to initiate delivery of the bursts of pulses to the first electrode for a first time duration, and to initiate delivery of the bursts of pulses to the second electrode for a second time duration.

4. The medical device of claim 1, wherein the control circuit is further configured to initiate delivery of the burst of pulses to the first electrode and simultaneously deliver the burst of pulses to the second electrode.

5. The medical device of claim 1, wherein the control circuit is further configured to change one or both of the first intra-burst period and the second intra-burst period of the pulses within a burst during the delivery of electrical neuromodulation energy.

6. The medical device of claim 1, wherein the control circuit is further configured to initiate delivery of a first burst of pulses to the first electrode using the first inter-burst period and simultaneously initiate delivery of the second burst of electrical pulses to the second electrode using a second inter-burst period.

7. The medical device of any one of claims 1-6, wherein the control circuit is further configured to modulate an amplitude and a pulse width of pulses of the bursts of pulses of electrical neurostimulation energy.

8. The medical device of claim 1, wherein the control circuit is further configured to:

   schedule delivery of pulses of electrical neurostimulation energy to the plurality of the implantable electrodes; and
   initiate delivery of a pulse of the scheduled pulses according to a probability function.

9. The medical device of claim 8, wherein the probability function comprises a Poisson process.

10. The medical device of claim 8, wherein the probability function comprises a randomization function.

11. The medical device of claim 8, wherein the probability function comprises a Gamma process.

12. The medical device of claim 8, wherein the probability function comprises a probability determined using a history of pulse delivery during a time period preceding the scheduled pulse delivery.

13. The medical device of claim 8, wherein the control circuit is further configured to:

schedule delivery of the pulses using a variable time interval between successive pulses; and
select a time interval for the variable time interval between successive pulses according to the probability function.

14. The medical device of claim 8, wherein the control circuit is further configured to:

schedule delivery of the pulses using a variable time interval between successive pulses; and
select a time interval for the variable time interval between successive pulses according to a randomization function.

15. The medical device of any one of claims 8-14,
wherein the control circuit is further configured to determine a number of pulses to include in the burst of pulses according to the probability function.

## Patentansprüche

1. Medizinprodukt (400) zur Kopplung mit mehreren implantierbaren Elektroden, wobei das Medizinprodukt aufweist:

eine Therapieschaltung (402), die so konfiguriert ist, dass sie elektrische Neurostimulationsenergie zu den mehreren implantierbaren Elektroden (26) abgibt; und
eine Steuerschaltung (404), die mit der Therapieschaltung betriebswirksam gekoppelt und so konfiguriert ist, dass sie:

die Abgabe von Impulsbursts der elektrischen Neurostimulationsenergie zu einer ersten Elektrode der mehreren implantierbaren Elektroden initiiert, wobei die der ersten Elektrode zugeführten Impulsbursts eine erste Inter-Burstperiode zwischen Bursts aufweisen und Impulse in einem der ersten Elektrode zugeführten Burst eine erste Intra-Burstperiode aufweisen; und
die Abgabe von Impulsbursts zu einer zweiten Elektrode der mehreren Elektroden initiiert, wobei die der zweiten Elektrode zugeführten Impulsbursts eine zweite Inter-Burstperiode zwischen Bursts aufweisen; und
**dadurch gekennzeichnet, dass** die Impulse in einem der zweiten Elektrode zugeführten Burst eine zweite Intra-Burstperiode aufweisen, die kleiner als die erste Intra-Burstperiode ist.

2. Medizinprodukt nach Anspruch 1, das ferner eine oder mehrere implantierbare Leitungen (12) aufweist, wobei die mehreren implantierbaren Elektroden an unterschiedlichen Positionen auf der einen oder den mehreren implantierbaren Leitungen angeordnet sind; und wobei die erste Elektrode und die zweite Elektrode unterschiedlichen Positionen auf der einen oder den mehreren implantierbaren Leitungen entsprechen, zu denen die Bursts abgegeben werden.

3. Medizinprodukt nach Anspruch 2, wobei die Steuerschaltung ferner so konfiguriert ist, dass sie die Abgabe der Impulsbursts zur ersten Elektrode für eine erste Zeitdauer initiiert und die Abgabe der Impulsbursts zur zweiten Elektrode für eine zweite Zeitdauer initiiert.

4. Medizinprodukt nach Anspruch 1, wobei die Steuerschaltung ferner so konfiguriert ist, dass sie die Abgabe der Impulsbursts zur ersten Elektrode initiiert und gleichzeitig die Impulsbursts zur zweiten Elektrode abgibt.

5. Medizinprodukt nach Anspruch 1, wobei die Steuerschaltung ferner so konfiguriert ist, dass sie die erste Intra-Burstperiode und/oder die zweite Intra-Burstperiode der Impulse in einem Burst während der Abgabe elektrischer Neurostimulationsenergie ändert.

6. Medizinprodukt nach Anspruch 1, wobei die Steuerschaltung ferner so konfiguriert ist, dass sie die Abgabe eines ersten Impulsbursts zur ersten Elektrode mit Hilfe der ersten Inter-Burstperiode initiiert und gleichzeitig die Abgabe des zweiten Bursts elektrischer Impulse zur zweiten Elektrode mit Hilfe einer zweiten Inter-Burstperiode initiiert.

7. Medizinprodukt nach einem der Ansprüche 1 bis 6, wobei die Steuerschaltung ferner so konfiguriert ist, dass sie eine Amplitude und eine Impulsbreite von Impulsen der Impulsbursts elektrischer Neurostimulationsenergie moduliert.

8. Medizinprodukt nach Anspruch 1, wobei die Steuerschaltung ferner so konfiguriert ist, dass sie:

   die Abgabe von Impulsen elektrischer Neurostimulationsenergie zu den mehreren implantierbaren Elektroden plant; und
   die Abgabe eines Impulses der geplanten Impulse gemäß einer Wahrscheinlichkeitsfunktion initiiert.

9. Medizinprodukt nach Anspruch 8, wobei die Wahrscheinlichkeitsfunktion einen Poisson-Prozess aufweist.

10. Medizinprodukt nach Anspruch 8, wobei die Wahrscheinlichkeitsfunktion eine Randomisierungsfunktion aufweist.

11. Medizinprodukt nach Anspruch 8, wobei die Wahrscheinlichkeitsfunktion einen Gamma-Prozess aufweist.

12. Medizinprodukt nach Anspruch 8, wobei die Wahrscheinlichkeitsfunktion eine Wahrscheinlichkeit aufweist, die mit Hilfe eines Impulsabgabeverlaufs während einer Zeitperiode bestimmt wird, die der geplanten Impulsabgabe vorausgeht.

13. Medizinprodukt nach Anspruch 8, wobei die Steuerschaltung ferner so konfiguriert ist, dass sie:

   die Abgabe der Impulse mit Hilfe eines variablen Zeitintervalls zwischen aufeinanderfolgenden Impulsen plant; und
   ein Zeitintervall für das variable Zeitintervall zwischen aufeinanderfolgenden Impulsen gemäß der Wahrscheinlichkeitsfunktion auswählt.

14. Medizinprodukt nach Anspruch 8, wobei die Steuerschaltung ferner so konfiguriert ist, dass sie:

   die Abgabe der Impulse mit Hilfe eines variablen Zeitintervalls zwischen aufeinanderfolgenden Impulsen plant; und
   ein Zeitintervall für das variable Zeitintervall zwischen aufeinanderfolgenden Impulsen gemäß einer Randomisierungsfunktion auswählt.

15. Medizinprodukt nach einem der Ansprüche 8 bis 14, wobei die Steuerschaltung ferner so konfiguriert ist, dass sie eine Anzahl von Impulsen, die zum Impulsburst gehören sollen, gemäß der Wahrscheinlichkeitsfunktion bestimmt.

**Revendications**

1. Dispositif médical (400) destiné à être couplé à une pluralité d'électrodes implantables, le dispositif médical comprenant :

   un circuit de thérapie (402) configuré pour fournir de l'énergie de neurostimulation électrique à la pluralité d'électrodes implantables (26) ; et
   un circuit de commande (404) couplé fonctionnellement au circuit de thérapie et configuré pour :

   initier la fourniture de salves d'impulsions de l'énergie de neurostimulation électrique à une première électrode de la pluralité d'électrodes implantables, dans lequel les salves d'impulsions appliquées à la première électrode comportent une première période inter-salves entre les salves, et les impulsions dans une salve appliquée à la première électrode comportent une première période intra-salve ; et
   initier la fourniture de salves d'impulsions à une deuxième électrode de la pluralité d'électrodes, dans lequel les salves d'impulsions appliquées à la deuxième électrode comportent une deuxième période inter-salves entre les salves ; et **caractérisé en ce que** les impulsions dans une salve appliquée à la deuxième électrode comportent une deuxième période intra-salve qui est inférieure à la première période intra-salve.

2. Dispositif médical selon la revendication 1, comprenant en outre une ou plusieurs sondes implantables (12), dans lequel la pluralité d'électrodes implantables sont disposées à différentes positions sur les une ou plusieurs sondes implantables ; et dans lequel la première électrode et la deuxième électrode correspondent à différentes positions sur les une ou plusieurs sondes implantables auxquelles les salves sont fournies.

3. Dispositif médical selon la revendication 2, dans lequel le circuit de commande est en outre configuré pour initier la fourniture des salves d'impulsions à la première électrode pendant une première durée de temps, et pour initier la fourniture des salves d'impulsions à la deuxième électrode pendant une deuxième durée de temps.

4. Dispositif médical selon la revendication 1, dans lequel le circuit de commande est en outre configuré pour initier la fourniture de la salve d'impulsions à la première électrode et pour fournir simultanément la salve d'impulsions à la deuxième électrode.

5. Dispositif médical selon la revendication 1, dans lequel le circuit de commande est en outre configuré pour modifier l'une ou l'autre ou les deux parmi la première période intra-salve et la deuxième période intra-salve des impulsions dans une salve pendant la fourniture d'énergie de neuromodulation électrique.

6. Dispositif médical selon la revendication 1, dans lequel le circuit de commande est en outre configuré pour initier la fourniture d'une première salve d'impulsions à la première électrode en utilisant la première période inter-salves, et pour initier simultanément la fourniture de la deuxième salve d'impulsions électriques à la deuxième électrode en utilisant une deuxième période inter-salves.

7. Dispositif médical selon l'une quelconque des revendications 1 à 6, dans lequel le circuit de commande est en outre configuré pour moduler une amplitude et une largeur d'impulsion des impulsions des salves d'impulsions d'énergie de neurostimulation électrique.

8. Dispositif médical selon la revendication 1, dans lequel le circuit de commande est en outre configuré pour :

planifier la fourniture d'impulsions d'énergie de neurostimulation électrique à la pluralité d'électrodes implantables ; et
initier la fourniture d'une impulsion des impulsions planifiées selon une fonction de probabilité.

9. Dispositif médical selon la revendication 8, dans lequel la fonction de probabilité comprend un processus de Poisson.

10. Dispositif médical selon la revendication 8, dans lequel la fonction de probabilité comprend une fonction de répartition aléatoire.

11. Dispositif médical selon la revendication 8, dans lequel la fonction de probabilité comprend un processus Gamma.

12. Dispositif médical selon la revendication 8, dans lequel la fonction de probabilité comprend une probabilité déterminée en utilisant un historique de fourniture d'impulsion pendant une période de temps précédant la fourniture d'impulsion planifiée.

13. Dispositif médical selon la revendication 8, dans lequel le circuit de commande est en outre configuré pour :

planifier la fourniture des impulsions en utilisant un intervalle de temps variable entre des impulsions successives ; et
sélectionner un intervalle de temps pour l'intervalle de temps variable entre des impulsions successives selon la fonction de probabilité.

14. Dispositif médical selon la revendication 8, dans lequel le circuit de commande est en outre configuré pour :

planifier la fourniture des impulsions en utilisant un intervalle de temps variable entre des impulsions successives ; et

sélectionner un intervalle de temps pour l'intervalle de temps variable entre des impulsions successives selon une fonction de répartition aléatoire.

15. Dispositif médical selon l'une quelconque des revendications 8 à 14, dans lequel le circuit de commande est en outre configuré pour déterminer un nombre d'impulsions à inclure dans la salve d'impulsions selon la fonction de probabilité.

**FIG. 1**

**FIG. 2**

**FIG. 3A**

**FIG. 3B**

**FIG. 3C**

**FIG. 3D**

*FIG. 3E*

*FIG. 3F*

*FIG. 3G*

*FIG. 3H*

FIG. 4

FIG. 5

**FIG. 6**

*FIG. 7*

*FIG. 8*

*FIG. 9*

**FIG. 10**

**FIG. 11**

**EP 3 568 196 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120095524 A1
- DE 102012002437 A1
- US 6895280 B
- US 6181969 B
- US 6516227 B
- US 6609029 B
- US 6609032 B
- US 6741892 B
- US 7949395 B
- US 7244150 B
- US 7672734 B
- US 7761165 B
- US 7974706 B
- US 8175710 B
- US 8224450 B
- US 8364278 B
- US 8700178 B
- US 8473061 B
- US 8571665 B
- US 8792993 B
- US 20100268298
- US 20110005069
- US 20110130803
- US 20110130816
- US 20110130817
- US 20110130818
- US 20110078900
- US 20110238129
- US 20120016378
- US 20120046710
- US 20120071949
- US 20120165911
- US 2012197375
- US 20120203316
- US 20120203320
- US 20120203321
- US 20130197424
- US 20130197602
- US 20140039587
- US 20140353001
- US 20140358208
- US 20140358209
- US 20140358210
- US 20150045864
- US 20150066120
- US 20150018915
- US 20150051681
- US 20150151113
- US 20140358207
- US 62113291
- US 20120197375